# EUROPÄISCHE PATENTSCHRIFT

(11) **EP 2 362 530 B1**
(45) Veröffentlichungstag und Bekanntmachung des Hinweises auf die Patenterteilung: **16.09.2015**
(21) Anmeldenummer: 11155952.2
(22) Anmeldetag: 25.02.2011
(51) Int. Cl.: H02K 41/03

(54) **Linearmotor mit permanentmagnetischer Selbsthaltung**
Linear motor with permanent magnetic lock
Moteur linéaire doté d'une auto-alimentation à aimant permanent

(30) Priorität: 26.02.2010 DE 102010000582
(43) Veröffentlichungstag der Anmeldung: 31.08.2011
(73) Patentinhaber: Karl Storz GmbH & Co. KG, 78532 Tuttlingen (DE)
(72) Erfinder: Vogel, Walter, 78647 Trossingen (DE)
(74) Vertreter: Lohr, Georg

(56) Entgegenhaltungen:
- EP-A2- 0 580 117
- WO-A1-99/18649
- WO-A2-2007/067704
- DE-A1- 10 323 629
- DE-A1-102006 006 877
- JP-A- 11 155 274
- JP-U- 54 121 207
- US-A- 4 071 042
- US-A- 4 127 835
- US-A- 5 896 076
- US-A1- 2006 208 600

## Beschreibung

### Technisches Gebiet

Die Erfindung betrifft einen Linearmotor insbesondere zum Einsatz in Endoskopen. Bei modernen Videoendoskopen sind ein Kamerachip sowie ein zugehöriges Linsensystem in die Endoskopspitze integriert. Zur Einstellung der Brennweite bzw. des Fokus des Linsensystems wird ein miniaturisierter Motor benötigt.

### Stand der Technik

Klassische Endoskope, wie sie beispielsweise für die minimalinvasive Chirurgie eingesetzt werden können, führen das Bild mittels Stablinsen vom intrakorporalen Objektiv zum extrakorporalen Okular. Durch die Stablinsen ist das System starr und in der optischen Qualität begrenzt. Moderne Videoendoskope verwenden einen Kamerachip in der Endoskopspitze. Ein solches Endoskop ist in der US 7,365,768 B1 offenbart. Dieses hat vor dem Kamerachip eine starr angeordnete Linse. Eine Einstellung der Brennweite der Linse ist nicht möglich.

Die DE 196 18 355 C2 zeigt einen in Endoskope integrierbaren Linearantrieb zum Einstellen der Brennweite eines Linsensystems. Dazu wird ein Permanentmagnet als Läufer innerhalb einer Statorspule bewegt. Der Linearantrieb ist wegen der großen Masse des Permanentmagneten jedoch träge. Der Zusammenhang zwischen dem Spulenstrom und der Läuferposition ist nicht eindeutig und macht einen zusätzlichen Wegsensor mit Lageregelung notwendig.

Die DE 37 17 872 C2 offenbart einen Antrieb mit einem Läufer und einem Stator für ein Linsensystem in Videokameras. Der Läufer besteht aus zwei Eisenhülsen, welche durch einen Träger zur Aufnahme des Linsensystems miteinander verbunden sind. Der Stator hat zwei Spulen sowie einen einzigen ringförmigen Permanentmagneten zur Erzeugung der für die Bewegung notwendigen Magnetfelder zwischen den Spulen. Der komplexe Aufbau des Antriebs kann bei Videokameras mit Linsendurchmessern im Zentimeterbereich gut realisiert werden, ist aber nicht auf die für endoskopische Anwendungen benötigte Größe im Millimeterbereich skalierbar.

In der DE 103 23 629 A1 ist ein Wanderfeld-Linearmotor entsprechend dem Oberbegriff des Anspruchs 1 offenbart, der mindestens drei Statorspulen aufweist. Durch eine phasenverschobene Bestromung der Spulen wird ein Wanderfeld erzeugt, welches eine Verschiebung des Läufers mit axialen Permanentmagneten bewirkt. Zur Erzeugung des Wanderfeldes wir eine aufwendige Ansteuerschaltung benötigt.

Aus der DE 10 2008 038 926 Al ist ein Linearantrieb bekannt, der zwei axial polarisierte Permanentmagnete im Läufer aufweist. Der Läufer wird durch die Bestromung der Statorspulen in axialer. Richtung ausgelehkt. Zusätzlich werden die stabilen Positionen des Läufers durch die im Stator angebrachten Polschuhe realisiert, so dass eine kontinuierliche Verschiebung des Läufers in einem Hüllrohr ermöglicht wird. Nachteillg ist dabei die Abhängigkeit des Hubs und der Stellkräfte von den weichmagnetischen Stator-Polschuhen, wodurch eine hohe Genauigkeit bei der Fertigung und der Montage dieser Teile vorausgesetzt wird.

### Darstellung der Erfindung

Der Erfindung liegt die Aufgabe zugrunde, einen Linearmotor mit derart kleinen Abmessungen zu schaffen, dass dieser in Endoskopen eingesetzt werden kann. Weiterhin soll der Linearmotor eine definierte Nullpunktlage sowie eine reproduzierbare Auslenkung in Abhängigkeit vom Ansteuersignal aufweisen. Zudem

In der EP 0 580 117 A2 wird ein Aktuator für elektronische Steuerungseinrichtungen offenbart, der eine zentrale Mittelachse mit daran befestigten Permanentmagneten sowie diese umgebende Spulen aufweist.

In der DE 10 2006 006877 A1 ist eine magnetische Antriebsvorrichtung zum Erzeugen einer oszillierenden mechanischen Bewegung oder zum Einstellen einer Position offenbart. Am Läufer befinden sich auf einer zentralen Achse Permanentmagnete. Der Stator hat eine Spule, welche mit einem Mantel aus ferromagnetischem Material umschlossen ist.

Die US 2006/208600 A1 zeigt einen elektromagnetischen Motor zur Erzeugung niederfrequenter Vibrationen. Es ist hier ein Läufer mit einem Permanentmagneten radial von zwei Spulen und seitlich von einem Permanentmagneten umschlossen.

Die US 5,896,076 A offenbart einen Aktuator mit einem Permanentmagneten im Läufer, sowie Spulen im Stator, wobei der Läufer durch Federn abgestützt wird.

Weiterhin soll der Linearmotor eine definierte Nullpunktlage sowie eine reproduzierbare Auslenkung in Abhängigkeit vom Ansteuersignal aufweisen. Zudem soll der Linearmotor große Antriebskräfte bei kleiner Masse aufweisen und somit eine schnelle, kontinuierliche und exakte Positionierung des optischen Systems in einem möglichst großen Bereich ermöglichen. Dabei darf der Strahlengang durch das optische System beim Verschieben der Komponenten nicht blockiert werden. Die Verlustleistung des Linearmotors soll gering sein, so dass wenig Wärme in der Spitze eines Endoskops entsteht. Für eine einfache Fertigung und Montage soll der Antrieb aus möglichst wenigen und geometrisch einfachen Einzelbauteilen zusammengesetzt sein.

Diese Aufgabe ist durch einen Linearmotor nach Anspruch 1 gelöst. Weiterbildungen des Linearmotors sind in den abhängigen Ansprüchen angegeben.

Der erfindungsgemäße Linearmotor umfasst einen Stator sowie einen dazu linear verschiebbaren Läufer. Der Stator hat mindestens eine Spule sowie an der Außenseite ein Rückschlußelement, welches zumindest weitgehend parallel zur Bewegungsrichtung angeordnet ist. Weiterhin ist am Stator mindestens ein axial magnetisierter Permanentmagnet vorgesehen. Die wenigstens eine Spule ist in einer Ebene mit dem wenigstens einen Permanentmagneten angeordnet. Der Begriff der Ebene bezieht sich im Falle einer zylindrischen Anordnung auf eine Zylinderfläche. Der Läufer wird von der mindestens einen Spule zumindest teilweise radial umschlossen und hat mindestens einen Permanentmagneten sowie mindestens einen Polschuh. Dabei durchdringt der magnetische Fluss des mindestens einen Permanentmagneten über die Polschuhe die wenigstens eine Spule. Vorteilhafterweise ist das Feld der Permanentmagneten im Stator parallel zum Feld der Permanentmagneten im Läufer, wobei sich die beiden Felder durchdringen. In Ruhestellung, also ohne Bestromung, des Linearmotors befindet sich jeder Polschuh innerhalb einer Spule, bevorzugt in Spulenmitte. Im Falle mehrerer nebeneinander liegender Spulen sind benachbarte Spulen jeweils in entgegengesetzter Richtung von Strom durchflossen. Der Läufer selbst weist einen Durchbruch oder eine Bohrung in axialer Richtung auf, worin beispielsweise ein optisches Element aufgenommen werden kann.

Die Besonderheit dieses Antriebs liegt in der Einbringung von Permanentmagneten nicht nur in den Läufer sondern zusätzlich auch in den Stator. Hiermit lassen sich hohe magnetische Flussdichten erzeugen und insbesondere dient ihre Anordnung der Einstellung einer stabilen axialen Mittelposition des Läufers (vergleich-bar zu einer zentrierenden mechanischen Feder). Durch Bestromung der Spulen wird der Läufer ausgelenkt, entgegen einer zunehmenden axialen Rückstellkraft durch die Magnetanordnung in Stator und Läufer. Hierdurch wird eine stromproportionale Auslenkung des Läufers ermöglicht.

In einer besonders vorteilhaften Ausgestaltung der Erfindung umfasst der Stator eine erste Spule sowie daneben angeordnet eine zweite Spule. Beide Spulen werden in entgegengesetzter Richtung von Strom durch-flossen d.h. bestromt. Weiterhin sind außerhalb der beiden Spulen neben der ersten Spule ein erster Permanentmagnet und neben der zweiten Spule ein zweiter Permanentmagnet angeordnet. Die beiden Permanentmagnete sind axial und gleichsinnig magnetisiert. Der Läufer hat einen ersten Permanentmagneten, welcher gegensinnig zu den Permanentmagneten des Stators magnetisiert ist. Weiterhin ist der erste Permanentmagnet des Läufers an beiden Seiten mit jeweils einem Polschuh verbunden.

Eine weitere vorteilhafte Ausführungsform dieser Ausgestaltung der Erfindung umfasst einen zusätzlichen ersten Polschuh im Stator. Dieser ist bevorzugt mittig zwischen den beiden Spulen angeordnet.

In einer weiteren vorteilhaften Ausgestaltung der Erfindung hat der Stator eine erste Spule sowie daneben angeordnet eine zweite Spule. Beide Spulen werden entgegengesetzt vom Strom durchflossen. Zwischen der ersten Spule und der zweiten Spule ist ein Permanentmagnet angeordnet. Der Läufer hat einen ersten Permanentmagneten, welcher gegensinnig zu dem Permanentmagneten des Stators magnetisiert ist. Weiterhin ist der erste Permanentmagnet des Läufers an beiden Seiten mit jeweils einem Polschuh verbunden.

In einer weiteren vorteilhaften Ausführungsform dieser Ausgestaltung der Erfindung hat der Stator zusätzlich einen ersten Polschuh neben der ersten Spule und einen zweiten Polschuh neben der zweiten Spule.

Eine weitere vorteilhafte Ausgestaltung der Erfindung betrifft einen Linearmotor mit einem Stator mit einer Spule und auf beiden Seiten der Spule angeordneten gegensinnig magnetisierten Permanentmagneten. Der Läufer hat zwei ebenfalls gegenseitig magnetisierte Permanentmagnete, zwischen denen ein Polschuh angeordnet ist. Die Magnetisierung der Permanentmagnete von Läufer und Stator ist ebenfalls gegensinnig ausgerichtet, so dass jeweils Nordpole oder auch Südpole zwischen Läufer und Stator gegenüber liegen.

Eine andere Ausgestaltung der Erfindung betrifft einen Linearmotor mit einem Stator mit drei Spulen, wobei die Spulen jeweils wechselseitig entgegengesetzt mit Strom durchflossen werden. Wird die mittlere Spule mit Strom in einer ersten Richtung durchflossen, so werden die beiden seitlich von dieser angeordneten Spulen mit Strom in entgegengesetzter Richtung durchflossen. Auf dieser Seite dieser Spulenanordnung ist ein Permanentmagnet angeordnet, wobei die beiden Permanentmagnete entgegengesetzt magnetisiert sind. Der Läufer weist zwei Permanentmagnete auf, die ebenfalls entgegengesetzt magnetisiert sind. Die Magnetisierung der Permanentmagnete von Läufer und Stator ist ebenfalls gegen-sinnig ausgerichtet, so dass jeweils Nordpole oder auch Südpole zwischen Läufer und Stator gegenüber liegen. Weiterhin ist zwischen den beiden Permanentmagneten ein Polschuh und auf den beiden freien Seiten der Permanentmagnete ebenfalls jeweils ein Polschuh angebracht.

Die verschiedenen Varianten der Erfindung sind bevorzugt spiegelsymmetrisch zu einer senkrecht auf der Mittelachse stehenden Ebene ausgeführt. Dies betrifft nicht die Magnetisierungsrichtungen der Permanentmagnete. Diese sind entsprechend ihrer Funktion ausgerichtet. Durch die spiegelsymmetrische Anordnung ergeben sich eine symmetrische Strom-Weg-Kennlinie und eine stabile Nullpunktlage des Läufers in der Mitte der Anordnung.

Die Länge des Rückschlußelements ist länger als die Länge des Läufers. Besonders vorteilhaft ist es, wenn die Länge des Rückschlußelements größer als die Länge des Läufers zuzüglich des maximalen Weges des Läufers ist.

Ein erfindungsgemäßer Linearmotor hat bevorzugt einen rotationssymmetrischen Läufer und/oder einen rotations-symmetrischen Stator. Bevorzugt ist der Linearmotor rotationssymmetrisch mit ringförmigen Rückschlußelement, Polschuhen, Permanentmagneten sowie einer ringförmigen Spule ausgeführt. Der Läufer und insbesondere die Permanentmagnete sowie die Polschuhe sind bevorzugt hohlzylindrisch, d.h. sie haben die Form einer zylindrischen Hülse. Der Strahlengang eines optischen Systems kann dann durch die Hülse verlaufen. Insbesondere kann eine Linse oder ein anderes optisches Element in der Hülse sitzen. Somit kann durch eine Verschiebung der Hülse die Brennweite und/oder auch der Fokus des optischen Systems eingestellt werden.

Der Linearmotor ermöglicht zwischen zwei Endstellungen eine exakte Einstellung der Position des Läufers relativ zum Stator. Bei dem Linearmotor entspricht jedem Spulenstrom eine eindeutige Position des Läufers in Bezug zum Stator. Somit kann durch eine Einstellung des Spulenstromes der Läufer in dem Verfahrbereich kontinuierlich verschoben werden. Durch diese eindeutige Zuordnung zwischen dem Spulenstrom und der Läuferposition kann auf die nach dem Stand der Technik notwendige Wegmessung zur Positionsbestimmung des Läufers verzichtet werden. Die Einzelbauteile haben eine einfache Geometrie (Ringe, Hülsen) und sind deshalb einfach herstell- und montierbar.

Die Polschuhe und das Rückschlußelement müssen immer ferromagnetische und/oder weichmagnetische Materialien umfassen.

Der Linearmotor ist bis zu einer Größe von wenigen Millimetern Außendurchmesser problemlos miniaturisier-bar. Die Verfahrstrecke zwischen den beiden Endstellungen des Läufers liegt bei einem Motor mit wenigen Millimetern Außendurchmesser typischerweise bei etwa 1 bis 3 mm.

Die Spule kann wahlweise auf einen Spulenkörper oder auch ohne Spulenkörper gewickelt sein. Sie kann auch mehrteilig sein, d.h. aus mehreren Wicklungen bestehen.

Eine detaillierte Darstellung der Funktion des Motors findet sich in der Beschreibung der Figuren.

In einer weiteren vorteilhaften Ausgestaltung der Erfindung ist zwischen dem Stator und dem Läufer eine Gleitschicht. Diese kann insbesondere im Falle einer rotationssymmetrischen Anordnung als Gleithülse ausgeführt sein. Um die Magnetfelder möglichst wenig zu beeinflussen soll die Gleitschicht aus einem nicht magnetfeldführenden Material, insbesondere aus einem nicht- ferromagnetischen Material bestehen. Ihre Ober-fläche umfasst bevorzugt ein Material mit niedrigem Reibungskoeffizienten, beispielsweise PTFE (Polytetrafluorethylen), Siliziumnitrid, Siliziumkarbid, poly-para-Xylylen Polymere oder DLC (diamond like carbon) wie beispielsweise in der US 5,478,650 offenbart.

Die Gleitschicht kann Unebenheiten auf der dem Läufer zugewandten Seite des Stators ausgleichen.

Ein erfindungsgemäßer Linearmotor kann auch aus Vollmaterial bestehen und an einem Ende einen Stößel zur Nanopositionierung von Instrumenten aufweisen. Eine solche Vorrichtung ist bevorzugt in der Molekularbiologie, der Mikroelektronik oder auch der Neurochirurgie einsetzbar.

Besonders günstig ist es, wenn die Spule (9) mit einem Gleichstrom mit überlagertem Wechselstrom kleiner Amplitude und einer Frequenz bis maximal 1kHz gespeist wird. Dadurch kann die Haft- und die Gleitreibung reduziert werden.

Ein weiterer Aspekt der Erfindung ist ein Verfahren zum Betrieb eines Linearmotors, wobei der Linearmotors mit einem Gleichstrom und einem überlagertem Wechselstrom kleiner Amplitude und einer Frequenz bis maximal 1kHz gespeist wird. Dadurch kann die Haftreibung beziehungsweise Gleitreibung im inneren des Motors reduziert werden.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt schematisch einen Linearmotor entsprechend der Erfindung.
Figur 2 zeigt eine Variante des Linearmotors mit einem zusätzlichen Polschuh im Stator.
Figur 3 zeigt eine weitere Variante des Linearmotors mit einem Permanentmagneten zwischen den Spulen.
Figur 4 zeigt eine Variante des Linearmotors aus Figur drei, wobei zusätzliche Polschuhe am Stator angeordnet sind.
Figur 5 zeigt eine weitere Variante des Linearmotors mit nur einer Spule.
Figur 6 zeigt eine weitere Variante der Erfindung mit drei Spulen.
Figur 7 zeigt den Linearmotor aus Figur 1 mit einer Linse.
Figur 8 zeigt den Feldverlauf im Linearmotor mit bestromter Spule.
Figur 9 zeigt Kraft-Weg Kennlinien des Linearmotors bei unterschiedlichen Spulenströmen
Figur 10 zeigt Kraft-Weg Kennlinien des Linearmotors mit und ohne Polschuh im Stator
Figur 11 zeigt die Feldverteilung eines Linearmotors nach Figur 3 im unbestromten Zustand.

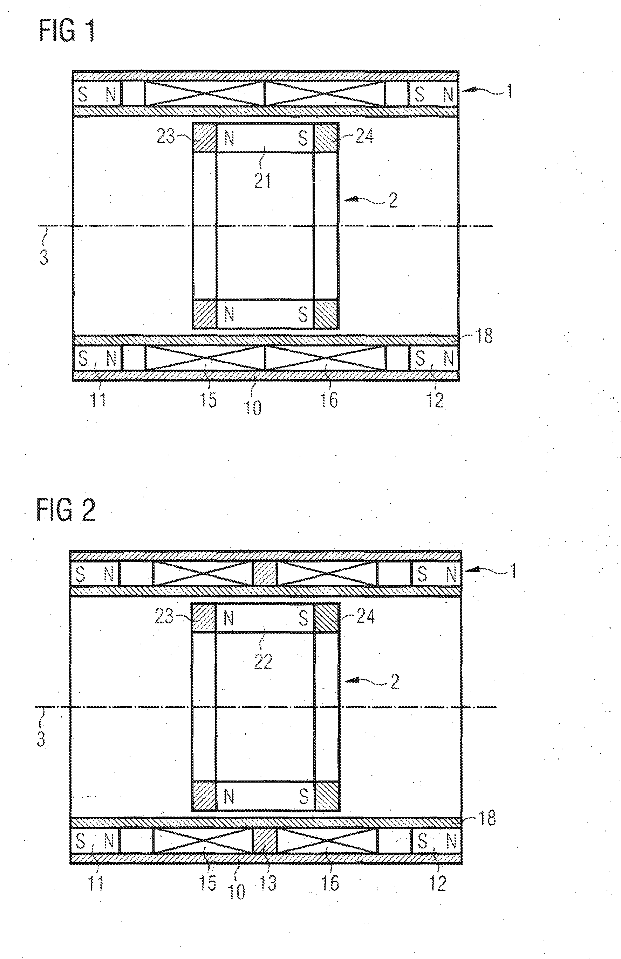

Figur 1 zeigt schematisch ein Ausführungsbeispiel einer erfindungsgemäßen Anordnung in zylindrischer Bauform im Schnitt. Der Stator 1 umfasst ein Rückschlußelement 10 in Form eines weichmagnetischen Rückschlussrohrs, in dessen Bohrung eine erste Spule 15 sowie eine zweite Spule 16 angeordnet sind. Auf einer Seite der Spulen ist ein erster Permanentmagnet 11 angeordnet. Auf der anderen Seite der Spulen ist ein zweiter Permanentmagnet 12 angeordnet. Beide Permanentmagnete sind axial gleichsinnig, dass heißt in der gleichen Richtung magnetisiert. Eine Gleitschicht 18 in Form einer Gleit-hülse schließt hier den Stator 9 nach innen ab und stellt eine reibungsarme Oberfläche für den Läufer zur Verfügung. Die Gleithülse muss aus einem nicht- ferromagnetischen Material bestehen. Der Läufer 2 umfasst hier einen Permanentmagnet 21 und einen ersten Polschuh 23 an der Seite des Nordpols des Permanentmagneten sowie einen zweiten Polschuh 24 an der Seite des Südpols. Der Läufer ist derart ausgerichtet, dass der Nordpol des Magneten 21 dem Nordpol des Permanentmagneten 11 im Stator gegenüber steht. Entsprechend steht dann der Südpol des Magneten 21 dem Südpol des Permanentmagneten 12 im Stator gegenüber. Dadurch ergibt sich eine abstoßende Reluktanzkraft in beiden Richtungen auf den Läufer und somit eine stabile Mittelpunktlage in der Mitte zwischen den beiden Permanentmagneten 11, 12 des Stators. In die Bohrung des Läufers kann ein zu positionierendes Element, wie eine optische Komponente, eingebracht werden. Der Läufer ist axial in beide Richtungen innerhalb der Gleithülse verschiebbar. Die Mittelachse 3 ist bei rotationssymmetrisch aufgebauten Anordnungen auch die Rotationsachse.

Figur 2 zeigt beispielhaft eine weitere Variante der Erfindung. Im Stator ist zwischen der ersten Spule 15 und der zweiten Spule 16 ein erster Polschuh 13 angeordnet.

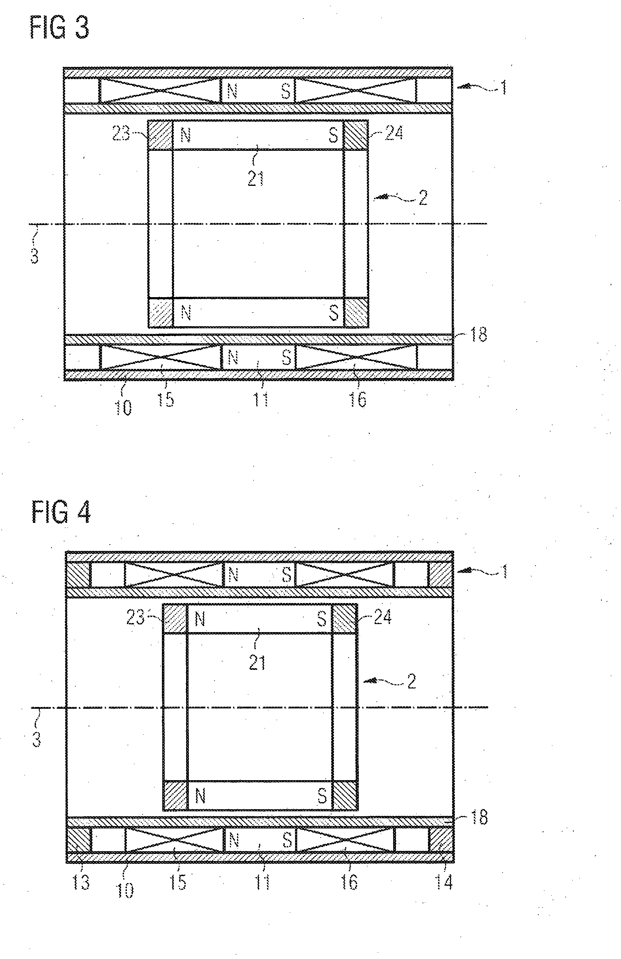

Figur 3 zeigt eine weitere Variante der Erfindung. Hier ist am Stator nur ein einziger Permanentmagnet 11 vorgesehen. Dieser ist zwischen der ersten Spule 15 und der zweiten Spule 16 angeordnet.

Figur 4 zeigt eine weitere Variante des Linearmotors nach Figur drei. Hierbei sind zusätzliche Polschuhe 13, 14 seitlich außerhalb der Spulen 15 und 16 angeordnet.

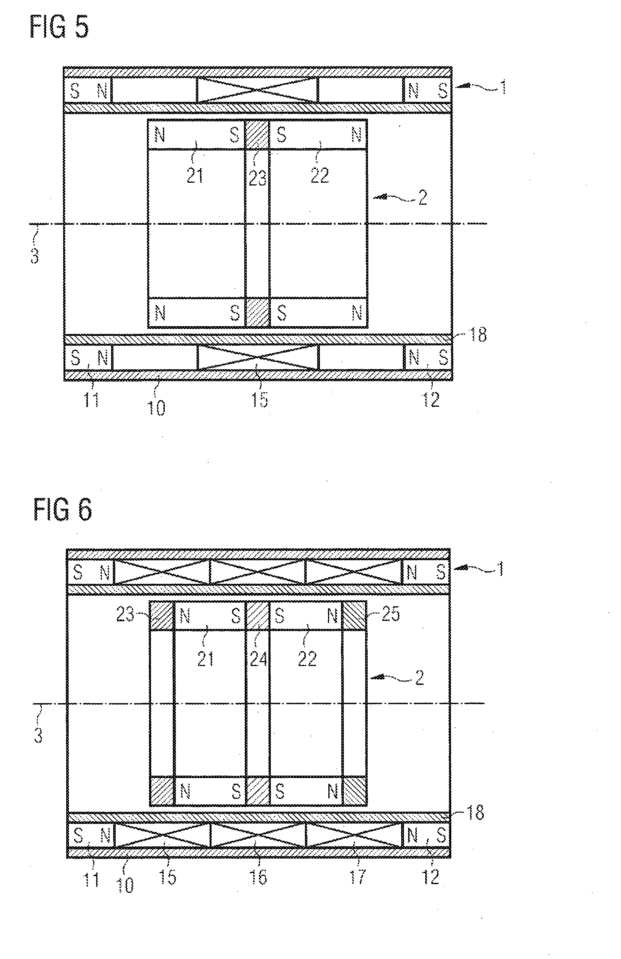

In Figur 5 ist eine weitere Variante des Linearmotors mit nur einer Spule dargestellt. Hier weist der Stator nur eine Spule 15 mit zwei seitlich von der Spule angeordneten Permanentmagneten 11, 12 auf. Die beiden Permanentmagnete 11, 12 sind in Axialrichtung entgegen-gesetzt magnetisiert. Der Läufer weist in der Mitte einen ersten Polschuh 23 auf an dem seitlich jeweils ein erster Permanentmagnet 21 und ein zweiter Permanentmagnet 22 angeordnet sind. Die beiden Permanentmagnete sind ebenfalls axial entgegengesetzt magnetisiert. In diesem Beispiel liegen die beiden Südpole der Permanentmagnete 21 und 22 an dem ersten Polschuh 23. Die Permanentmagnete 11, 12 des Stators sind axial entgegengesetzt zu den entsprechenden Magneten des Läufers magnetisiert.

In Figur 6 ist eine weitere Variante der Erfindung mit drei Spulen offenbart. Hier weist der Stator eine erste Spule 15, eine zweite Spule 16 und eine dritte Spule 17 nebeneinander liegend auf. Seitlich neben den Spulen sind ein erster Permanentmagnet 11 und ein zweiter Permanentmagnet 12 axial entgegengesetzt magnetisiert angeordnet. Der Läufer hat drei Polschuhe 23, 24 und 25. Zwischen dem ersten Polschuh 23 und dem zweiten Polschuh 24 ist ein erster Permanentmagnet 21 angeordnet. Ein zweiter Permanentmagnet 22 befindet sich zwischen dem zweiten Polschuh 24 und dem dritten Polschuh 25. Die Magnetisierung der Permanentmagnete ist wie in der Anordnung nach Figur 5. Durch die höhere Spulenanzahl können hier die Stellkräfte vergrößert und die Positioniergenauigkeit erhöht werden.

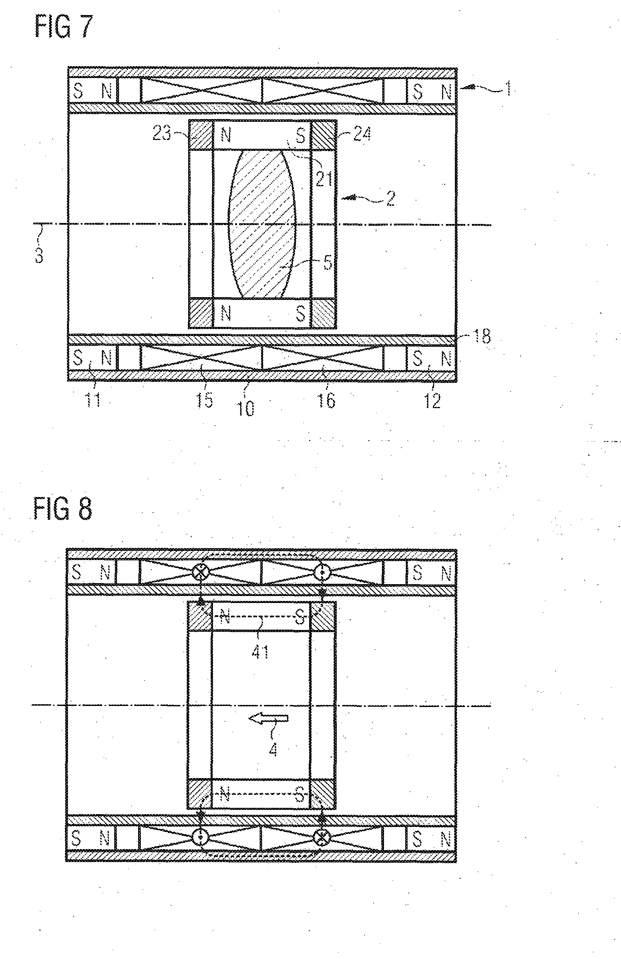

Figur 7 zeigt einen Linearmotor nach Figur 1, wobei noch eine Linse 5 dargestellt ist, die durch den Linearmotor in Richtung der Mittelachse 15 verschoben werden kann.

Figur 8 zeigt eine Darstellung der Erfindung mit den magnetischen Kreisen bei bestromten Spulen. Es wird hier ein Strom wie durch die Richtungspfeile angezeigt, durch die Spulen geleitet, so dass in der Spule 15 in der oberen Hälfte der Figur der Strom in die Zeichnungsebene hinein fließt und entsprechend in der unteren Hälfte der Figur aus der Zeichnungsebene heraus fließt. Der Stromfluss durch die Spule 16 erfolgt in umgekehrter Richtung. Der magnetische Fluss vom Permanentmagneten 21 über die beiden Polschuhe 23 und 24 sowie das Rückschlußelement 10 durchdringt die Spulen 15 und 16. Durch einen Stromfluss durch die Spulen wird eine Lorentzkraft in Bewegungsrichtung 4 auf den Läufer erzeugt. Diese wirkt der Reluktanzkraft zwischen dem Permanentmagneten 21 des Läufers und den Permanentmagneten 11, 12 des Stators entgegen.

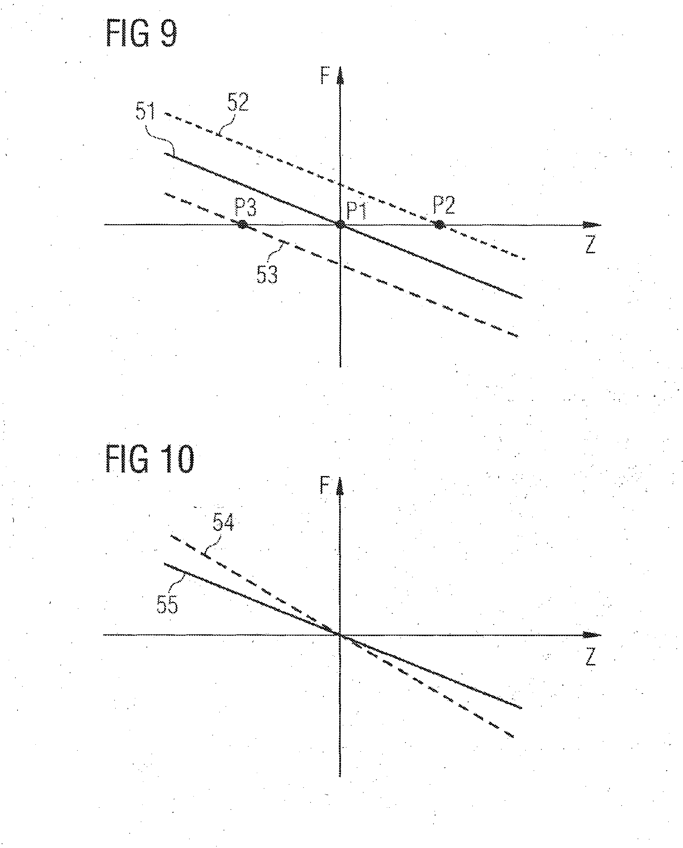

Figur 9 zeigt die Kraft-Weg-Kennlinien bei verschiedenen Spulenströmen. In dem Diagramm ist die Kraft F auf den Läufer als Funktion der Auslenkung z des Läufers dargestellt. Eine positive Kraft F ist nach oben und eine positive Auslenkung z nach rechts aufgetragen. Die Kurve 51 zeigt die Kraft-Weg-Kennlinie bei unbestromter Spule, das heißt bei einem Spulenstrom null. Bei einer positiven Auslenkung z des Läufers, das heißt in z-Richtung nach rechts ergibt sich durch die Reluktanzkräfte eine negative Rückstellkraft F, die versucht, den Läufer an seiner Position zu halten. Die Rückstellkraft ist in der Position P1 gleich null. Somit wird der Läufer in diesem Punkt ohne Bestromung der Spule eine stabile Lage einnehmen. Wird der Läufer in Folge extern wirkender Kräfte, beispielsweise Beschleunigungskräfte, in eine seiner beiden Bewegungsrichtungen aus dieser stabilen Position heraus gedrängt, so wirken rücktreibende Kräfte, die den Läufer in die zentrale Position zurückdrängen. Die magnetischen Kräfte halten den Läufer vergleichbar zu einer mechanischen Feder in dieser Position.

Mit einem Spulenstrom in einer ersten Richtung wird den Reluktanzkräften eine zusätzliche Lorentzkraft überlagert. Somit verschiebt sich die Kurve 51 in Richtung der Kurve 52. Daraus resultiert in dem Punkt P2, der in positiver z-Richtung von dem Punkt P1 entfernt liegt, die Rückstellkraft null, so dass der Läufer eine neue stabile Lage in diesem Punkt einnimmt. Entsprechendes geschieht bei einem entgegengesetzt gepolten Strom, jedoch in umgekehrter Richtung. Es wird die Kurve 51 in Richtung der Kurve 53 verschoben. Der Punkt P3 mit der Rückstellkraft null liegt nun in der entgegengesetzten Richtung des ursprünglichen Punktes P1.

Es wird hier der Begriff des Spulenstroms verwendet. Dieser ist unabhängig von der Anzahl der in der jeweiligen Variante der Erfindung vorgesehenen Spulen. Selbstverständlich werden benachbarte Spulen mit Strömen entgegengesetzter Richtung durchflossen. Bei einer Umkehrung der Polarität kehrt sich somit auch die Polarität der Ströme durch alle Spulen um. Der Begriff der Stromstärke bezieht sich auf den Strom durch eine Spule.

Figur 10 zeigt die Auswirkung eines Polschuhs 13, 14 im Stator anhand von Kraft-Weg-Kennlinien. Die Kennlinien beziehen sich auf unbestromte Spulen. Die Darstellung zeigt grundsätzlich wie in der vorhergehenden Figur die Funktion der Rückstellkraft F auf den Läufer als Funktion der Auslenkung z. Die Kurve 54 entsteht bei einer typischen Anordnung entsprechend der Erfindung. Wird nun ein zusätzlicher Polschuh 13, 14 in den Stator integriert, so ergibt sich eine Verringerung der rück-stellenden Reluktanzkräfte und somit eine flachere Kennlinie. Somit lässt sich durch den Einbau von zusätzlichen Polschuhen und selbstverständlich auch durch deren Dimensionierung die Kennlinie in weiten Bereichen einstellen. Ein Vorteil einer flacheren Kennlinie ist beispielsweise, dass zur Betätigung des Läufers ein niedrigerer Spulenstrom benötigt wird, welcher wiederum zu einer niedrigeren Verlustleistung in den Spulen führt. Gerade bei endoskopischen Instrumenten ist es wünschenswert, die Verlustleistung in der Spitze des Instruments möglichst gering zu halten, um den Ort der Untersuchung möglichst wenig zu beeinflussen. Hier bietet der Einsatz eines zusätzlichen Polschuhs ein einfaches Optimierungsinstrument.

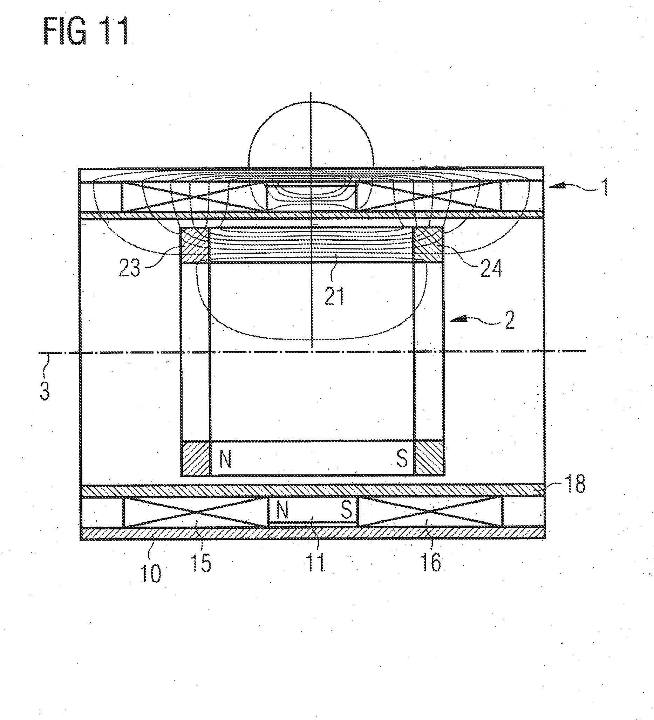

Figur 11 zeigt die Feldverteilung eines Linearmotors nach Figur 3 im unbestromten Zustand. Hierbei sind die magnetischen Feldlinien in die obere Hälfte einer Schnittdarstellung nach Figur 3 eingezeichnet. Tatsächlich ist das Feld wie die gesamte Anordnung rotations-symmetrisch.

### Bezugszeichenliste

- 1: Stator
- 2: Läufer
- 3: Mittelachse
- 4: Bewegungsrichtung
- 5: Linse
- 10: Rückschlußelement
- 11: Erster Permanentmagnet im Stator
- 12: Zweiter Permanentmagnet im Stator
- 13: Erster Polschuh im Stator
- 14: Zweiter Polschuh im Stator
- 15: Erste Spule
- 16: Zweite Spule
- 17: Dritte Spule
- 18: Gleitschicht
- 21: Erster Permanentmagnet im Läufer
- 22: Zweiter Permanentmagnet im Läufer
- 23: Erster Polschuh im Läufer
- 24: Zweiter Polschuh im Läufer
- 25: Dritter Polschuh im Läufer
- 31: Erster magnetischer Kreis
- 41: Erster Elektromagnetischer Kreis
- 51: Kraft-Weg-Kennlinie stromlos
- 52: Kraft-Weg-Kennlinie bei Strom in einer ersten Richtung
- 53: Kraft-Weg-Kennlinie bei Strom in einer zweiten Richtung
- 54: Kraft-Weg-Kennlinie ohne Polschuh im Stator
- 55: Kraft-Weg-Kennlinie mit Polschuh im Stator
- F: Kraft auf den Läufer
- z: Auslenkung des Läufers
- P1: Position des Läufers im stromlosen Zustand
- P2: Position des Läufers mit Strom in einer ersten Richtung
- P3: Position des Läufers mit Strom in einer zweiten Richtung

## Patentansprüche

1. Linearmotor zum Einsatz in Endoskopen umfassend
einen Stator (1) mit wenigstens einer Spule (15, 16, 17), und
einem von der wenigstens einen Spule radial umschlossenen, parallel zum Stator verschiebbaren Läufer (2) mit einen Durchbruch oder einer Bohrung in axialer Richtung und mit wenigstens einem axial magnetisierten Permanentmagneten (21, 22) und einer Anzahl Polschuhen (23, 24, 25), wobei der magnetische Fluss des wenigstens einen Permanentmagneten des Läufers (2) über die Polschuhe die wenigstens eine Spule durchdringt, und wobei ohne Bestromung des Linearmotors jeder Polschuh sich Innerhalb einer Spule befindet,
**dadurch gekennzeichnet, dass**
der Stator (1) wenigstens einen axial magnetisierten Permanentmagneten (11, 12), welche von einem Rückschlußelement (10) umschlossen sind, umfasst,
die Anzahl der Spulen der Anzahl der Polschuhe (23, 24, 25) entspricht und die wenigstens eine Spule (15, 16, 17) auf einer Zylindermantelfläche mit dem wenigstens einen Permanentmagneten (11, 12) des Stators (1) angeordnet ist.

2. Linearmotor nach Anspruch 1,
**dadurch gekennzeichnet, dass**
der Stator (1) eine erste Spule (15) sowie eine daneben angeordnete zweite Spule (16) umfasst, wobei die erste Spule (15) in entgegengesetzter Richtung wie die zweite Spule (16) bestromt wird, und dass neben der ersten Spule (15) ein erster Permanentmagnet (11) sowie neben der zweiten Spule (16) ein zweiter Permanentmagnet (12) vorgesehen Ist, wobei die beiden Permanentmagnete gleichsinnig magnetisiert sind, und
der Läufer einen ersten Permanentmagnet (21) auf-weist, welcher entgegengesetzt zu den Permanentmagneten (11, 12) des Stators magnetisiert ist und an jedem Ende mit einem Polschuh (23, 24) verbunden ist.

3. Linearmotor nach Anspruch 2,
**dadurch gekennzeichnet, dass**
der Stator einen ersten Polschuh (13) aufweist, der zwischen der ersten Spule (15) und der zweiten Spule (16) angeordnet ist.

4. Linearmotor nach Anspruch 1,
**dadurch gekennzeichnet, dass**
der Stator (1) eine erste Spule (15) sowie eine daneben angeordnete zweite Spule (16) umfasst, wobei die erste Spule (15) in entgegengesetzter Richtung wie die zweite Spule (16) bestromt wird, und dass zwischen der ersten Spule (15) sowie der zweiten Spule (16) ein erster Permanentmagnet (11) vorgesehen ist, und
der Läufer einen ersten Permanentmagneten (21) aufweist, welcher gleichsinnig zu dem ersten Permanentmagneten (11) des Stators magnetisiert ist und an jedem Ende mit einem Polschuh (23, 24) verbunden ist.

5. Linearmotor nach Anspruch 4,
**dadurch gekennzeichnet, dass**
der Stator einen ersten Polschuh (13) neben der ersten Spule (15) und einen zweiten Polschuh (14) neben der zweiten Spule (16) aufweist.

6. Linearmotor nach Anspruch 1,
**dadurch gekennzeichnet, dass**
der Stator (1) eine erste Spule (15) umfasst, und dass neben der ersten Spule (15) ein erster Permanentmagnet (11) sowie neben der zweiten Spule (16) ein zweiter Permanentmagnet (12) vorgesehen ist, wobei die beiden Permanentmagnete gegensinnig magnetisiert sind, und der Läufer einen ersten Permanentmagneten (21) auf der Seite des ersten Permanentmagneten (11) des Stators, der gegensinnig zum ersten Permanentmagneten (11) des Stators magnetisiert ist sowie einen zweiten Permanentmagneten (22) auf der Seite des Zweiten Permanentmagneten (12) des Stators aufweist, der gegensinnig zum Zweiten Permanentmagneten (12) des Stators magnetisiert ist, wobei zwischen dem ersten Permanentmagneten (21) des Läufers und dem zweiten Permanentmagneten (23) des Läufers ein erster Polschuh (23) angeordnet ist.

7. Linearmotor nach Anspruch 1,
**dadurch gekennzeichnet, dass**
der Stator (1) eine erste Spule (15), eine daneben angeordnete zweite Spule (16) sowie eine weiter daneben angeordnete Dritte Spule (17) umfasst, wo-bei die erste Spule (15) in gleicher Richtung wie die dritte Spule (17) und beide in entgegengesetzter Richtung wie die zweite Spule (16) bestromt werden, und dass neben der ersten Spule (15) ein erster Permanentmagnet (11) sowie neben der dritten Spule (17) ein zweiter Permanentmagnet (12) vorgesehen ist, wobei die beiden Permanentmagnete gegensinnig magnetisiert sind, und
der Läufer einen ersten Permanentmagneten (21) auf der Seite des ersten Permanentmagneten (11) des Stators, der gegensinnig zum ersten Permanentmagneten (11) des Stators magnetisiert ist sowie einen zweiten Permanentmagneten (22) auf der Seite des Zweiten Permanentmagneten (12) des Stators aufweist, der gegensinnig zum Zweiten Permanentmagneten (12) des Stators magnetisiert ist, wobei auf einer Seite des ersten Permanentmagneten (21) ein erster Polschuh (23), zwischen dem ersten Permanentmagneten (21) des Läufers und dem zweiten Permanentmagneten (23) des Läufers ein zweiter Polschuh (24) und auf einer Seite des zweiten Permanentmagneten (22) ein dritter Polschuh (25) an-geordnet ist.

8. Linearmotor nach Anspruch 1 oder 2,
**dadurch gekennzeichnet, dass**
die Länge des Läufers (2) kleiner als die Länge des Rückschlusselementes (10) ist.

9. Linearmotor nach einem der vorhergehenden Ansprüche,
**dadurch gekennzeichnet, dass**
Im Inneren des Läufers (2) ein optisches Element (3) aufgenommen werden kann.

10. Linearmotor nach einem der vorhergehenden Ansprüche,
**dadurch gekennzeichnet, dass**
der Läufer rotationssymmetrisch ist.

11. Linearmotor nach einem der vorhergehenden Ansprüche,
**dadurch gekennzeichnet, dass**
der Stator rotationssymmetrisch ist.

12. Linearmotor nach einem der vorhergehenden Ansprüche,
**dadurch gekennzeichnet, dass**
eine Gleithülse (6) mit einem Material mit niedrigem Reibungskoeffizienten an der Oberfläche zwischen Stator (1) und Läufer (2) angeordnet ist.

13. Linearmotor nach einem der vorhergehenden Ansprüche,
**dadurch gekennzeichnet, dass**
der Läufer (2) aus Vollmaterial besteht und lediglich einen Stößel zur Nanopositionierung von Instrumenten aufweist.

14. Verfahren zum Betrieb eines Linearmotors nach einem der vorhergehenden Ansprüche,
**dadurch gekennzeichnet, dass**
zur Minderung der Haft- und Gleitreibung dem Gleichstrom durch die
Spule (9) ein sehr kleiner Wechselstrom mit Frequenzen bis maximal 1 kHz überlagert wird.

## Claims

1. Linear motor for use in endoscopes comprising
a stator (1) having at least one coil (15, 16, 17) and
a slider (2) being radially enclosed by the at least one coil and being slideable parallel to the stator, with an aperature or a bore in axial direction and with at least one axially magnetized permanent magnet (21, 22), and a plurality of pole shoes (23, 24, 25), wherein the magnetic flux of the at least one permanent magnet of the slider (2) via the pole shoes penetrates the at least one coil, and wherein each pole shoe is disposed inside a coil in the absence of energization of the linear motor;
**characterized in that**
the stator (1) comprises at least one axially magnetized permanent magnet (11, 12) being enclosed by a flux return element (10),
the number of coils corresponds to the number of pole shoes (23, 24, 25), and the at least one coil (15, 16, 17) is arranged on a cylindrical outer surface with the at least one permanent magnet (11, 12) of the stator (1),

2. Linear motor according to claim 1,
**characterized in that**
the stator (1) comprises a first coil (15) as well as an adjacently disposed second coil (16), wherein the first coil (15) is energized in opposite direction as the second coil (16), and that a first permanent magnet (11) is provided adjacent to the first coil (15), and a second permanent magnet (12) is provided adjacent to the second coil (16), wherein both permanent magnets are magnetized in the same direction, and the slider comprises a first permanent magnet (21) being magnetized in opposite direction to the permanent magnets (11, 12) of the stator and being connected to a pole shoe (23, 24) at each end, respectively.

3. Linear motor according to claim 2,
**characterized in that**
the stator comprises a first pole shoe (13) being disposed between the first coil (15) and the second coil (16).

4. Linear motor according to claim 1,
**characterized in that**
the stator (1) comprises a first coil (15) and an adjacently disposed second coil (16), wherein the first coil (15) is energized in opposite direction as the second coil (16), and wherein a first permanent magnet (11) is provided between the first coil (15) and the second coil (16), and
the slider comprises a first permanent magnet (21) being magnetized in the same direction as the first permanent magnet (11) of the stator and being connected to a pole shoe (23, 24) at each end, respectively.

5. Linear motor according to claim 4,
**characterized in that**
the stator (1) comprises a first pole shoe (13) adjacent to the first coil (15) and a second pole shoe (14) adjacent to the second coil (16).

6. Linear motor according to claim 1,
**characterized in that**
the stator (1) comprises a first coil (15) and that a first permanent magnet (11) is provided adjacent to the first coil (15), and a second permanent magnet (12) is provided adjacent to the second coil (16), wherein both permanent magnets are magnetized in opposite direction, and
the slider comprises a first permanent magnet (21) on the side of the first permanent magnet (11) of the stator being magnetized in opposite direction to the first permanent magnet (11) of the stator, and a second permanent magnet (22) on the side of second permanent magnet (12) of the stator being magnetized in opposite direction to the second permanent magnet (12) of the stator, wherein a first pole shoe (23) is arranged between the first permanent magnet (21) of the slider and the second permanent magnet (23) of the slider.

7. Linear motor according to claim 1,
**characterized in that**
the stator (1) comprises a first coil (15), an adjacently disposed second coil (16), and a further adjacently disposed third coil (17), wherein the first coil (15) is energized in the same direction as the third coil (17) and both the first coil (15) and the third coil (17) are energized in opposite direction as the second coil (16), and wherein a first permanent magnet (11) is provided adjacent to the first coil (15) and a second permanent magnet (12) is provided adjacent to the third coil (17), wherein both permanent magnets are magnetized in opposite direction, and
the slider comprises a first permanent magnet (21) on the side of the first permanent magnet (11) of the stator being magnetized in opposite direction to the first permanent magnet (11) of the stator, and comprises a second permanent magnet (22) on the side of the second permanent magnet (12) of the stator being magnetized in opposite direction as the second permanent magnet (12) of the stator, wherein a first pole shoe (23) is disposed on a side of the first permanent magnet (21), a second pole shoe (24) is disposed between the first permanent magnet (21) of the slider and the second permanent magnet (23) of the slider, and a third pole shoe (25) is disposed on a side of the second permanent magnet (22).

8. Linear motor according to claim 1 or 2,
**characterized in that**
the length of the slider (2) is smaller than the length of the flux return element (10).

9. Linear motor according to one of the preceding claims,
**characterized in that**
an optical element (3) can be received in the interior of the slider (2).

10. Linear motor according to one of the preceding claims,
**characterized in that**
the slider is rotationally symmetrical.

11. Linear motor according to one of the preceding claims,
**characterized in that**
the stator is rotationally symmetrical.

12. Linear motor according to one of the preceding claims,
**characterized in that**
a sliding sleeve (6) comprising a material with a low friction coefficient is arranged at the surface between the stator (1) and slider (2).

13. Linear motor according to one of the preceding claims,
**characterized in that**
the slider (2) consists of solid material and has only a plunger for nanopositioning of instruments.

14. A method for operating a linear motor according to any one of the preceding claims,
**characterized in that**
a very small alternating current with frequencies up to maximally 1 kHz is superimposed on the direct current through the coil (9), in order to reduce static and dynamic friction.

## Revendications

1. Moteur linéaire destiné à être utilisé dans des endoscopes, comprenant un stator (1) avec au moins une bobine (15, 16, 17) et un bloc mobile (2) entouré dans le sens radial par l'au moins une bobine et capable de translation parallèlement au stator avec une ouverture ou un perçage dans le sens axial et avec au moins un aimant permanent (21, 22) magnétisé dans le sens axial et avec un certain nombre de pièces polaires (23, 24, 25), le flux magnétique de l'au moins un aimant permanent du bloc mobile (2) pénétrant dans l'au moins une bobine en passant par les pièces polaires et chaque pièce polaire se trouvent à l'intérieur d'une bobine quand le moteur linéaire n'est pas sous tension, **caractérisé en ce que**
le stator (1) comprend au moins un aimant permanent (11, 12) magnétisé dans le sens axial qui est entouré par un élément de retour (10),
le nombre de bobines correspond au nombre de pièces polaires (23, 24, 25) et
l'au moins une bobine (15, 16, 17) est disposée sur une surface d'enveloppe cylindrique avec l'au moins un aimant permanent (11, 12) du stator (1).

2. Moteur linéaire selon la revendication 1, **caractérisé en ce que** le stator (1) comprend une première bobine (15) ainsi qu'une deuxième bobine (16) disposée à côté de celle-ci, la première bobine (15) étant parcourue par le courant dans le sens opposé de la deuxième bobine (16), et **en ce qu'**il est prévu à côté de la première bobine (15) un premier aimant permanent (11) et à côté de la deuxième bobine (16) un deuxième aimant permanent (12), les deux aimants permanents étant magnétisés dans le même sens, et
le bloc mobile présente un premier aimant permanent (21) qui est magnétisé dans le sens opposé des deux aimants permanents (11, 12) du stator et qui est relié à chaque extrémité à une pièce polaire (23, 24).

3. Moteur linéaire selon la revendication 2, **caractérisé en ce que** le stator présente une première pièce polaire (13) qui est disposée entre la première bobine (15) et la deuxième bobine (16).

4. Moteur linéaire selon la revendication 1, **caractérisé en ce que** le stator (1) comprend une première bobine (15) et une deuxième bobine (16) disposée à côté de celle-ci, la première bobine (15) étant parcourue par le courant dans le sens opposé de la deuxième bobine (16), et **en ce qu'**il est prévu entre la première bobine (15) et la deuxième bobine (16) un premier aimant permanent (11), et
le bloc mobile comprend un premier aimant permanent (21) qui est magnétisé dans le même sens que le premier aimant permanent (11) du stator et qui est relié à chaque extrémité à une pièce polaire (23, 24).

5. Moteur linéaire selon la revendication 4, **caractérisé en ce que** le stator comporte une première pièce polaire (13) à côté de la première bobine (15) et une deuxième pièce polaire (14) à côté de la deuxième bobine (16).

6. Moteur linéaire selon la revendication 1, **caractérisé en ce que** le stator (1) comprend une première bobine (15) et **en ce qu'**il est prévu à côté de la première bobine (15) un premier aimant permanent (11) et à côté de la deuxième bobine (16) un deuxième aimant permanent (12), les deux aimants permanents étant magnétisés dans des sens opposés, et
le bloc mobile comporte un premier aimant permanent (21) du côté du premier aimant permanent (11) du stator, qui est magnétisé dans le sens opposé du premier aimant permanent (11) du stator, ainsi qu'un deuxième aimant permanent (22) du côté du deuxième aimant permanent (12) du stator, qui est magnétisé dans le sens opposé du deuxième aimant permanent (12) du stator, une première pièce polaire (23) étant disposée entre le premier aimant permanent (21) du bloc mobile et le deuxième aimant permanent (23) du bloc mobile.

7. Moteur linéaire selon la revendication 1, **caractérisé en ce que** le stator (1) comporte une première bobine (15), une deuxième bobine (16) disposée à côté de celle-ci ainsi qu'une troisième bobine (17) disposée plus loin à côté, la première bobine (15) étant parcourue par le courant dans le même sens que la troisième bobine (17) et toutes deux étant parcourues par le courant dans le sens opposé de la deuxième bobine (16), et
**en ce qu'**il est prévu à côté de la première bobine (15) un premier aimant permanent (11) et à côté de la troisième bobine (17) un deuxième aimant permanent (12), les deux aimants permanents étant magnétisés dans des sens opposés, et
le bloc mobile comporte un premier aimant permanent (21) du côté du premier aimant permanent (11) du stator, qui est magnétisé dans le sens opposé du premier aimant permanent (11) du stator, ainsi qu'un deuxième aimant permanent (22) du côté du deuxième aimant permanent (12) du stator, qui est magnétisé dans le sens opposé du deuxième aimant permanent (12) du stator, une première pièce polaire (23) étant disposée sur un côté du premier aimant permanent (21), une deuxième pièce polaire (24) entre le premier aimant permanent (21) du bloc mobile et son deuxième aimant permanent (23) et une troisième pièce polaire (25) sur un côté du deuxième aimant permanent (22).

8. Moteur linéaire selon la revendication 1 ou 2, **caractérisé en ce que** la longueur du bloc mobile (2) est inférieure à la longueur de l'élément de retour (10).

9. Moteur linéaire selon l'une des revendications précédentes, **caractérisé en ce qu'**un élément optique (3) peut être logé à l'intérieur du bloc mobile (2).

10. Moteur linéaire selon l'une des revendications précédentes, **caractérisé en ce que** le bloc mobile est symétrique dans le sens de la rotation.

11. Moteur linéaire selon l'une des revendications précédentes, **caractérisé en ce que** le stator est symétrique dans le sens de la rotation.

12. Moteur linéaire selon l'une des revendications précédentes, **caractérisé en ce qu'**une bague glissante (6) faite d'un matériau à faible coefficient de frottement est disposée sur la surface entre le stator (1) et le bloc mobile (2).

13. Moteur linéaire selon l'une des revendications précédentes, **caractérisé en ce que** le bloc mobile (2) est fait d'un matériau plein et présente seulement un poussoir pour le nano-positionnement d'instruments.

14. Procédé pour l'utilisation d'un moteur linéaire selon l'une des revendications précédentes, **caractérisé en ce qu'**afin de réduire le frottement par adhérence et par glissement, un très faible courant alternatif, d'une fréquence inférieure ou égale à 1 kHz au maximum, est superposé au courant continu par la bobine (9).
